(19) Europäisches Patentamt
European Patent Office
Office européen des brevets

(11) EP 2 924 041 B1

(12) EUROPEAN PATENT SPECIFICATION

(45) Date of publication and mention of the grant of the patent:
**23.03.2016 Bulletin 2016/12**

(21) Application number: **15160712.4**

(22) Date of filing: **25.03.2015**

(51) Int Cl.:
*C07D 498/22* (2006.01) *G01N 33/00* (2006.01)
*G01N 21/00* (2006.01)

# (54) RIGID QUINOXALINE CAVITANDS AS MOLECULAR RECEPTORS FOR THE DETECTION OF AROMATIC COMPOUNDS IN THE AIR

RIGIDE CHINOXALIN CAVITANDS ALS MOLEKULARE REZEPTOREN FÜR DIE DETEKTION VON AROMATISCHEN VERBINDUNGEN IN DER LUFT

"CAVITANDS" RIGIDES FONCTIONALISÉS AVEC DES QUINOXALINES COMME RÉCEPTEURS MOLÉCULAIRES POUR LA DÉTECTION DES COMPOSÉS AROMATIQUES DANS L'AIR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2014 IT MI20140523**

(43) Date of publication of application:
**30.09.2015 Bulletin 2015/40**

(73) Proprietor: **Soatec S.r.l.**
**43126 Parma (IT)**

(72) Inventors:
- **Trzcinski, Jakub**
  **42-200 CZESTOCHOWA (PL)**
- **Pinalli, Roberta**
  **43036 FIDENZA (PARMA) (IT)**
- **Bianchi, Federica**
  **43124 PARMA (IT)**
- **Massera, Chiara**
  **43123 PARMA (IT)**
- **Dalcanale, Enrico**
  **43123 PARMA (IT)**

(74) Representative: **Biggi, Cristina**
**Bugnion S.p.A.**
**Viale Lancetti 17**
**20158 Milano (IT)**

(56) References cited:
- **FEDERICA BIANCHI ET AL: "Cavitands as superior sorbents for benzene detection at trace level", NEW JOURNAL OF CHEMISTRY, vol. 27, no. 3, 27 February 2003 (2003-02-27), pages 502-509, XP055142877, ISSN: 1144-0546, DOI: 10.1039/b210942e**
- **JENS HORNUNG ET AL: "Cycloalkane and Alicyclic Heterocycle Complexation by New Switchable Resorcin[4]arene-Based Container Molecules: NMR and ITC Binding Studies", CHEMISTRY - A EUROPEAN JOURNAL, vol. 17, no. 44, 24 October 2011 (2011-10-24), pages 12362-12371, XP055142994, ISSN: 0947-6539, DOI: 10.1002/chem.201101861**
- **GOTTSCHALK T ET AL: "Reversibly controllable guest binding in precisely defined cavities: selectivity, induced fit, and switching in novel resorcin[4]arene-based container molecules", TETRAHEDRON, ELSEVIER SCIENCE PUBLISHERS, AMSTERDAM, NL, vol. 64, no. 36, 1 September 2008 (2008-09-01), pages 8307-8317, XP023315791, ISSN: 0040-4020, DOI: 10.1016/J.TET.2008.04.102 [retrieved on 2008-04-30]**

EP 2 924 041 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present invention relates to the sector of volatile compound analysis through gas phase extraction (*solid-phase micro extraction* -SPME-, *purge&trap* and dynamic headspace technologies). In particular, the invention relates to a method for the selective adsorption and subsequent desorption and determination in the air of aromatic compounds belonging to the BTEX class (Benzene, Toluene, Ethyl benzene and Xylenes). This method envisages the use of SPME fibres or cartridges for *purge&trap* and dynamic headspace comprising a sensitive element able to recognise and specifically adsorb the analyte of interest.

In the case of the present invention the sensitive element is an organic compound generally called cavitand Qx, which acts as a coating of the SPME fibre or as an adsorbent element in adsorbent cartridges. In another aspect, the invention relates to new compounds, called cavitands, useful as sensitive elements for the adsorption and, therefore, selective pre-concentration of aromatic compounds in the air.

### Background art

Environmental pollution due to vehicle traffic and industrial production is a problem that is constantly increasing, which requires continuous widespread monitoring to guarantee healthy life conditions. Low cost systems are currently offered based on solid state gas sensors, such as Metal-Oxide Sensors (MOX). However, MOXs are lacking in selectivity for applications in which individual classes of analytes must be detected and quantified within complex matrices, such as ambient air. Furthermore, the responses of these sensors are particularly affected by humidity variation, which produces unwanted MOX sensor baseline drift.

To quantify atmospheric pollutants, some analytical techniques are still used, such as gas chromatography (GC). GC is very selective as is it able to separate individual analytes in complex gas mixtures. In stations for automatic air quality control, gas chromatography is used for the quantitation of aromatic volatile compounds, such as benzene, toluene, ethyl benzene and xylenes. GC columns separate the gaseous mixtures into the various compounds exploiting the different affinities for the stationary phase. In this way, thanks to the flow of the vector gas, the individual gaseous compounds elute from the column at different times.

In environmental analyses the specific detection of aromatic compounds is particularly important, being these substances generally toxic or carcinogenic (e.g. benzene) even at very low levels of concentration such as $ppb_V$ (Directive 2000/69/EC, Off. J. Eur. Communities, L 313/12, issued 13.12.2000 (http://eur-lex.europa.eu); A.T. Hodgson, H. Levin, Volatile Organic Compounds in Indoor Air: A Review of Concentrations Measured in North America Since 1990, Lawrence Berkeley National Laboratory, 2003, p. 31; Carcinogenic Effects of Benzene: An Update, EPA/600/P-97/001 F April 1998.).

A miniaturized GC system (mini GC) was previously produced based on microsystem technologies with three "micromachined core" elements: a pre-concentration unit, a GC separation column and a MOX-based detection unit (S. Zampolli, I. Elmi, F. Mancarella, P. Betti, E. Dalcanale, G.C. Cardinali, M. Severi, Sensors and Actuators B, 2009, 141, 322-328; S. Zampolli, P. Betti, I. Elmi, E. Dalcanale, Chem. Commun., 2007, 2790-2792). This mini GC is provided with a simplified injection system and a portable vector gas generator, which make it a portable and easily transportable system. The pre-concentration column comprises a micromachined silicon cartridge, containing a stationary phase, comprising quinoxaline cavitands (QxCav) represented in the appended Figure 1. The QxCav have a cavity 8.3 Å deep, able to selectively complex aromatic molecules (P. Soncini, S. Bonsignore, E. Dalcanale, F. Ugozzoli, J. Org. Chem., 1992, 57, 4608) through weak CH-$\pi$ and $\pi$-$\pi$ interactions that are established between the aromatic cavity walls and the analyte (M. Vincenti, E. Dalcanale, P. Soncini, G. Guglielmetti, J. Am. Chem. Soc., 1990, 112, 445; M. Vincenti, E. Pelizzetti, E. Dalcanale, P. Soncini, Pure Appl. Chem., 1993, 65, 1507; M. Vincenti, E. Dalcanale, J. Chem. Soc Perkin Trans. 2, 1995, 1069), while other interferents, such as aliphatic compounds and water, are not withheld. Details on the analytical abilities of QxCav can be found in literature (E. Dalcanale, G. Costantini, P. Soncini, J. Incl. Phenom. Mol Recognit. Chem., 1992, 13, 87; E. Dalcanale, J. Hartmann, Sens. Actuators B, 1995, 24, 39; F. Bianchi, R. Pinalli, F. Ugozzoli, S. Spera, M. Careri, E. Dalcanale, New J. Chem., 2003, 27, 502; E.B. Feresenbet, E. Dalcanale, C. Dulcey, D.K. Shenoy, Sens. Actuators B, 2004, 97, 211; S. Zampolli, P. Betti, I. Elmi, E. Dalcanale, Chem. Commun., 2007, 2790-2792; F. Bianchi, M. Mattarozzi, P. Betti, F. Bisceglie, M. Careri, A. Mangia, L. Sidisky, S. Ongarato, E. Dalcanale, Anal. Chem., 2008, 80, 6423). In order to be used as an adsorbent material in chromatographic analyses with *purge&trap* systems, the QxCav was crystallised from acetone, and sieved on 35-60 mesh molecular sieves, obtaining uniform particles of diameter 420-730 $\mu$m. This system was able to selectively detect benzene, toluene, ethyl benzene and xylenes.

The limit of the quinoxaline cavitand used as pre-concentration material in this monitoring system is its conformation flexibility, which reduces its complexing properties and hence its performance. In fact, this type of cavitand can

fluctuate from a Kite type conformation, with open aromatic walls, to a Vase type conformation, where the aromatic walls are parallel to one another (as shown in Figure 2). The shift from one conformation to another is influenced by changes in pH, temperature and the solvent. Diederich has performed detailed studies on the quinoxaline cavitand confirming its great flexibility both in solution and in the solid state (L. Shirtcliff, H. Xu, F. Diederich, Eur. J. Org. Chem., 2010, 846). In particular, in the solid state, the balance is strongly shifted towards the Vase conformation and the shift from one conformation to another is strongly dependent on temperature (Figure 3).

The ability of the quinoxaline cavitand of the prior art to pass from a Vase conformation to a Kite conformation according to the temperature represents a limit to its complexation ability and selectivity and, therefore, to detect pollutants in the air, particularly benzene, toluene, ethyl benzene and xylenes, which are particularly hazardous to human health and which, therefore, must be constantly monitored.

## Summary of the invention

The present invention aims to overcome the limits of quinoxaline cavitands of the prior art by providing new generation quinoxaline cavitands whose conformation has been blocked in the Vase position thanks to the introduction, on the upper edge of the cavity, of bridges that unite the quinoxaline rings. The stiffening of the cavity leads to the achievement of a stable Vase shape, no longer influenced by temperature variations and therefore to an increase in the complexation properties of the cavitand and therefore of the sensitivity and selectivity in detecting atmospheric pollutants in the air, such as Benzene, Toluene, Ethyl benzene, *o-, m-, p*-Xylene (BTEX).

One object of the present invention is to make the quinoxaline cavitand very sensitive and selective towards the aromatic compounds that belong to the BTEX family only.

A further object of the present invention is to use these cavitands for making fibres for solid phase microextraction or adsorbent systems for *purge&trap* and dynamic headspace, for example cartridges, to be used in the selective sampling of environmental pollutants such as Benzene, Toluene, Ethyl benzene, *o-, m-, p-X*ylene (BTEX).

A further object of the invention is to provide a method for monitoring environmental levels of Benzene, Toluene, Ethyl benzene, *o-, m-, p*-Xylene (BTEX) wherein an SPME fibre or a cartridge for *purge&trap*/dynamic headspace made with the cavitands according to the invention are used for the selective concentration/adsorption of the BTEX pollutants, which are subsequently desorbed and analysed using chromographic techniques, such as, for example GC-MS.

## Brief description of the drawings

Figure 1 shows the structural formula of the quinoxaline cavitands of the prior art;

Figure 2 shows the Vase and Kite conformations of the quinoxaline cavitands of the prior art;

Figure 3 shows the temperature dependency of conformation fluctuations of the known cavitands from Vase to Kite;

Figure 4 shows the selectivity results of the cavitands according to the invention QxBoxEt and QxBoxMe to a mixture of BTEX in air (Benzene 349 ng/$m^3$, Toluene 411 ng/$m^3$, Ethyl benzene 474 ng/$m^3$, *m- p- o*-Xylene 473 ng/$m^3$; SPME extraction, 15 min, RT);

Figure 5 shows the results of consecutive desorptions from 50°C to 250°C of a mixture of aliphatic hydrocarbons and BTEX in air with the cavitand QxBoxEt (pentane 32100 ng/$m^3$, hexane 38400 ng/$m^3$, heptane 44600 ng/$m^3$, octane 50900 ng/$m^3$, nonane 57100 ng/$m^3$, BTEX: 5-696 ng/$m^3$; SPME extraction, 15 min, RT);

Figure 6 shows the selectivity of the cavitand QxBoxMe to a mixture of BTEX in the air (Benzene: 70 ng/$m^3$, Toluene: 411 ng/$m^3$, Ethyl benzene: 95 ng/$m^3$, *m*-xylene + *p*-Xylene: 190 ng/$m^3$, *o*-Xylene: 95 ng/$m^3$; SPME extraction, 15 min, RT);

Figure 7 shows, at the top, the geometry of the cavitand QxBoxEt obtained by single crystal X-ray analysis; at the bottom, a detail of the cavity of the cavitand (the hydrogen atoms have been omitted for clarity);

Figure 8 shows the molecular structure of QxBoxEt@benzene (at the top) and QxBoxEt@toluene (at the bottom); in the coordination complex with toluene, only one of the two possible orientations is shown; only the hydrogen atoms of the analyte are represented;

Figure 9 shows, on the left, the geometry of the cavitand QxBoxMe obtained by single crystal X-ray analysis; on the right, a detail of the cavity (the hydrogen atoms have been omitted for clarity);

Figure 10 shows the molecular structure of QxBoxMe@benzene (at the top) and QxBoxMe@toluene (at the bottom); (only the hydrogen atoms of the analyte are represented).

## Detailed description of the invention

The objects of the invention described above are reached by the following compounds with the general formula:

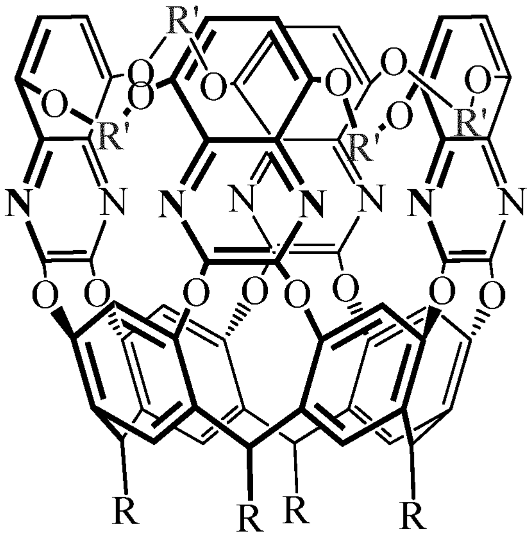

(I)

wherein

R' is chosen from $-CH_2-$ and $-CH_2-CH_2-$
R is a $C_1$-$C_{20}$ linear or branched alkyl chain, not substituted or substituted with a group chosen from: an olefin, acetylenic, alcoholic, azidic, phosphonic or acid group.

Preferably, R is a linear or branched alkyl chain with 6 carbon atoms. Preferably, the substituent group on the alkyl chain R is at an end position of the chain.

Further on in the description QxBoxMe will be used to indicate the cavitand according to the invention wherein R' is $-CH_2-$ and R is as described above, and QxBoxEt will be used to indicate the cavitand according to the invention wherein R' is $-CH_2-CH_2-$ and R is as described above.

In general, the cavitand QxBoxMe has higher complexation ability than QxBoxEt with respect to BTEX. The cavitand QxBoxEt reveals a higher selectivity to xylenes, whereas the cavitand QxBoxMe shows excellent complexation ability to benzene, as can be deduced from the results of the studies performed and inserted below.

The invention also relates to the use of the quinoxaline cavitands of formula (I) for preparing SPME fibres to be used for the selective microextraction (pre-concentration) of pollutants such as Benzene, Toluene, Ethyl benzene, *o-, m-, p*-Xylene from the air. The pollutants selectively extracted (pre-concentrated) are then desorbed and analysed with conventional systems, such as, for example, gas chromatography and mass spectrometry.

The invention also relates to the use of the cavitands of formula (I) for preparing cartridges for dynamic headspace or *purge&trap* to be used for specifically recognising and adsorbing (pre-concentrating) pollutants such as Benzene, Toluene, Ethyl benzene, *o-, m-*, *p*-Xylene from the air. The pollutants selectively extracted (pre-concentrated) are then desorbed from the cartridge and analysed with conventional systems, such as, for example, gas chromatography and mass spectrometry.

The SPME fibres were created by immersing the silicon support of the fibres in an epoxy glue resistant to high temperatures and then in the finely pulverised solid cavitand.

The cartridges for dynamic headspace and *purge&trap* were made by filling normal glass cartridges with an appropriate amount of pulverised cavitand, previously sieved for having particle size in the range 60-80 mesh.

The subject matter of the invention is also an SPME fibre made and/or coated with the quinoxaline cavitand of formula (I) and its use for the selective determination (adsorption or pre-concentration) of pollutants chosen from Benzene, Toluene, Ethyl benzene, *o-, m-*, *p*-Xylene in the air. The subject matter of the invention is also a cartridge for dynamic headspace or *purge&trap* made with the cavitand of formula (I) and its use for the selective determination (adsorption or pre-concentration) of pollutants chosen from Benzene, Toluene, Ethyl benzene, *o-, m-*, *p*-Xylene in the air.

The subject matter of the invention is also the use of the cavitands according to the invention for monitoring atmospheric pollution, in particular due to Benzene, Toluene, Ethyl benzene, *o-, m-*, *p*-Xylene. The method for determining the concentration of the pollutants comprises the selective adsorption of the BTEX pollutants in the cavity of the cavitands according to the invention and their subsequent desorption and quantitative analysis using gas chromatographic techniques, such as, for example GC-MS.

## Synthesis of QxBox cavitands

The preparation of the cavitands QxBoxMe I and QxBoxEt II is performed following the synthetic method indicated in the diagram below. The synthesis envisages the bridging of resorcinarene 1 (prepared according to the procedure reported in L. M. Tunstad, J. A. Tucker, E. Dalcanale, J. Weiser, J. A. Bryant, J. C. Sherman, R. C. Hegelson, C. B. Knobler, D.J. Cram, J. Org. Chem. 1989, 54, 1305-1312) with 4 dimethoxy-quinoxaline groups 2 (prepared according to the procedure reported in S. Oguchi, Bull. Chem. Soc. Jp. 1968, 41, 980-987). The product 3 thus obtained is deprotected for removal of the 8 methyl groups to provide 4, a common intermediate product for the preparation of both cavitands I and II. The final bridging is performed respectively with bromochloromethane for I and ethylene glycol ditosylate for II.

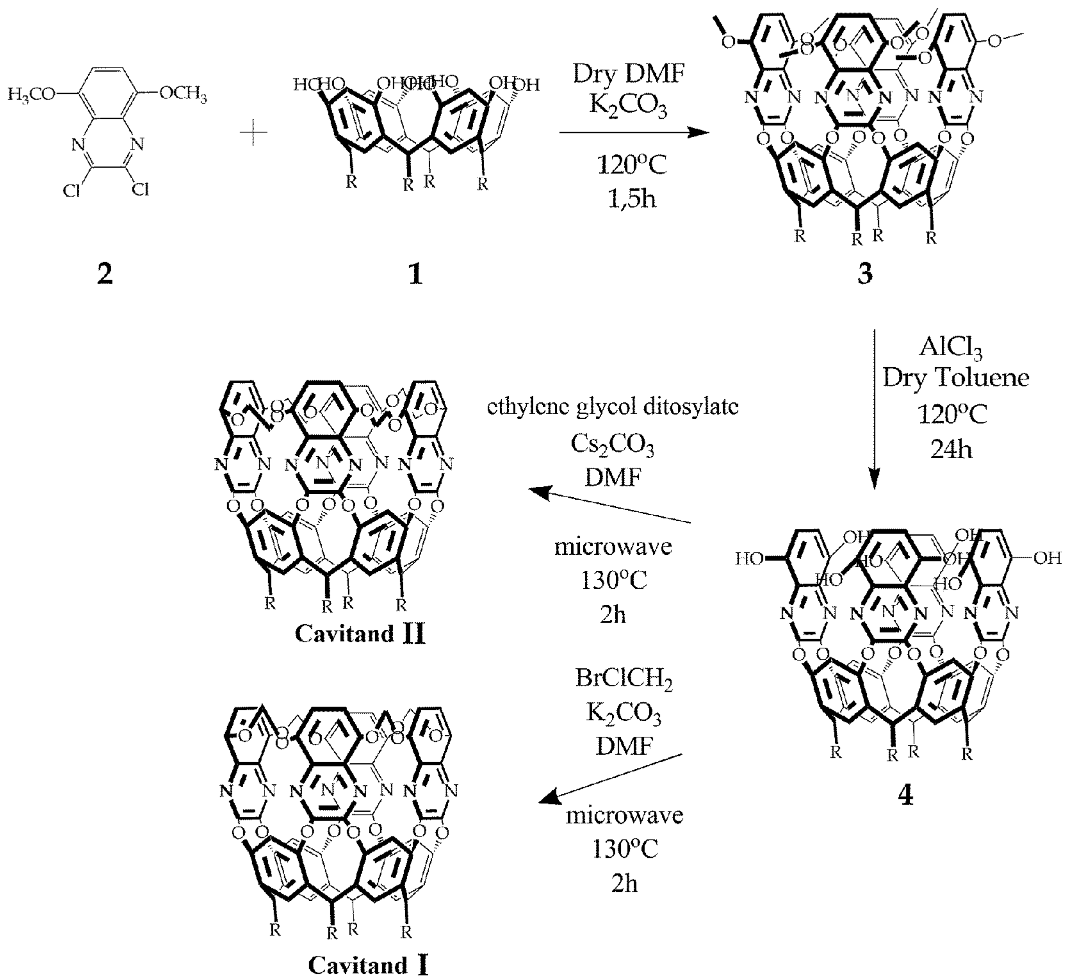


2 1 3

4

Cavitand II

Cavitand I

## SYNTHESIS OF THE OCTAMETHOXY QUINOXALINE CAVITAND ($R=C_6H_{13}$)

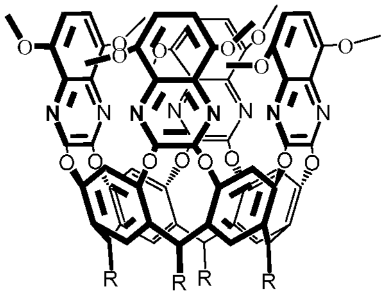

**3**

1,00 g (1,17.10$^{-3}$ mol) of resorcinarene 1 are dissolved in 40 ml of anhydrous and degassed DMF. In an atmosphere of Argon 2,60 g (18,78.10$^{-3}$ mol) of anhydrous $K_2CO_3$ are added and 1,20 g (4,70.10$^{-3}$ mol) of 2,3-dichloro-5,8-dimethoxyquinoxaline 2. The reaction is conducted in a Schlenk flask at 80°C for 16 hours. The DMF is removed through a vacuum trap. 50 ml of aqueous solution of acid (HCl 1 M), and 50 ml of dichloromethane are added. Through liquid/liquid separation the organic phase is recovered, which is dried in the rotovapor. The pure yellow-orange product is purified through column chromatography ($SiO_2$, gradient from $CH_2Cl_2$ 100% to acetone 100%, product eluent: $CH_2Cl_2$: acetone 9:1) and obtained with a yield of 50%.

**$^1$H-NMR** (300 MHz, $CDCl_3$): $\delta$ = 0.77 (t, 12H, *J* = 6.2 Hz, $\mathbf{CH_3}CH_2CH_2$), 1.10-1.25 (m, 32H, **$-CH_2$-),** 1.97 (q, 8H, *J* = 6.8 Hz, $CH\mathbf{CH_2}CH_2$), 3.92 (t, 4H, *J* = 7.4 Hz, $\mathbf{CH}CH_2CH_2$), 3.98 (s, 24H, $\mathbf{CH_3O}Ar$), 6.8 (s, 4H, $Ar\mathbf{H}_{down}$), 6.90 (s, 8H, $CH_3OAr\mathbf{H_2}$) 7.37 (s, 4H, $Ar\mathbf{H}_{up}$)

**MALDI MS:** m/z 1569,71 $[M]^+$

**SYNTHESIS OF THE OCTAHYDROXY QUINOXALINE CAVITAND (R=$C_6H_{13}$)**

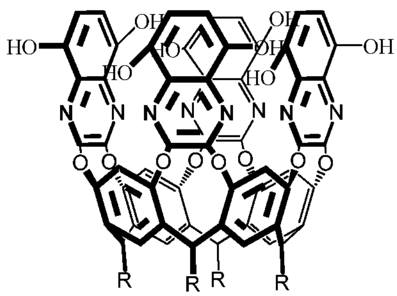

4

The cavitand 4 is obtained through the deprotection of the oxygens of the methoxy groups of the cavitand 3. 1,00 g (0,64.10$^{-3}$ mol) of cavitand 3 are dissolved in 40 ml of anhydrous toluene. 0,850 g (6,37-10$^{-3}$ mol) of anhydrous $AlCl_3$ are added to the solution. The reaction mixture is shaken at 130°C for 24 hours.

The reaction is stopped by adding water at 130°C; it is cooled to room temperature maintaining magnetic agitation. The product is then purified through liquid/liquid separation (toluene/$H_2O$). The organic phase is recovered, dried and the solid obtained is sonicated in ethyl ether and dried in the rotovapor. The solid is then suspended in hexane and filtered. The filtrate is sonicated again with ethyl ether and filtered. The product obtained is a pale yellow crystalline powder. The yield of the reaction is 85%.

**$^1$H-NMR** (400 MHz, DMSO-$d_6$): $\delta$ = 0.79 (t, 12H, *J* = 6.6 Hz, $\mathbf{CH_3}CH_2CH_2$), 1.14-1.35 (m, 32H, -$\mathbf{CH_2}$-), 2.33 (bq, 8H $CH\mathbf{CH_2}CH_2$ ), 4.62 (bt, 4H, $\mathbf{CH}CH_2CH_2$), , 6.94 (s, 8H, $CH_3OAr\mathbf{H_2}$), 7.51 (s, 4H, $Ar\mathbf{H}_{down}$), 7.77 (s, 4H, $Ar\mathbf{H}_{up}$), 9.21 (s, 8H, ArO**H**)

**MALDI MS:** m/z 1457,48 $[M+H]^+$, 1479,47 $[M+Na]^+$

**SYNTHESIS OF THE CAVITAND I-QxBoxMe con R=$C_6H_{13}$**

The cavitand of formula I wherein R=$C_6H_{13}$ was synthesized with the following procedure:

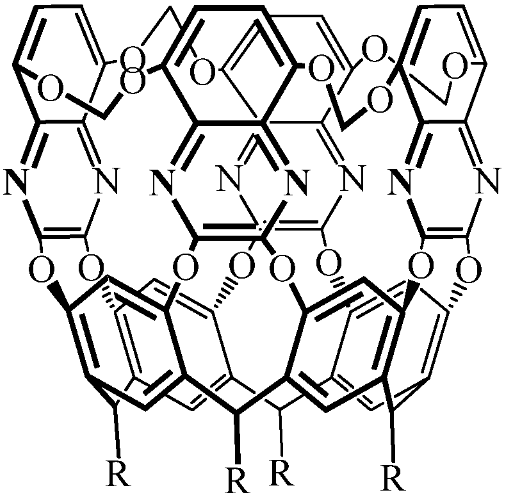

I

In a microwave test tube in an Argon atmosphere 100 mg (6,49.$10^{-5}$ mol; 1 eq.) of cavitand 4 are suspended in 7 ml of distilled DMF. After sonication, 126 mg (9,08.$10^{-4}$ mol; 14 eq.) of anhydrous $K_2CO_3$ and 126 μl (1,95.$10^{-3}$ mol; 30 eq.) of bromochloromethane are added to the suspension. The reaction is conducted in a microwave reactor by irradiating the mixture at 100 W with magnetic agitation for 90 min, maintaining a temperature of 120°C. Once the reaction has taken place, the crude product is dried by means of a vacuum pump; the crude product obtained is solubilized with 10 ml of dichloromethane and washed with $H_2O$ (3x10ml). The organic phase is recovered and dried. The product is purified through preparative flash column chromatography ($SiO_2$, gradient from $CH_2Cl_2$ 100% to acetone 100%, product eluent: $CH_2Cl_2$: acetone 95:5). The product obtained is a yellow crystalline powder. The yield of the reaction is 5%.
**$^1$H-NMR** (300 MHz, acetone-*$d_6$*): δ = 0.88 (t, 12H, *J* = 6.7 Hz, **$CH_3$**$CH_2CH_2$), 1.21-1.47 (m, 32H, -**$CH_2$-**), 2.52 (bq, 8H CH**$CH_2$**$CH_2$), 5.76 (t, 4H, *J* = 7.5 Hz, **CH**$CH_2CH_2$), 6.20 (d, 4H, *J* = 6.1 Hz, ArO**$CH_2$**O), 6.60 (d, 4H, *J* = 6.1 Hz, ArO**$CH_2$**O), 7.32 (s, 8H, Ar**$H_2$**), 7.94 (s, 4H, Ar**$H_{down}$**), 8.13 (s, 4H, Ar**$H_{up}$**)
**MALDI TOF-TOF:** calculated for $C_{88}H_{80}N_8O_{16}$ [M] m/z= 1504.5692, obtained m/z= 1504.2636 $[M]^+$

**SYNTHESIS OF THE CAVITAND II-QxBoxEt con R=$C_6H_{13}$**

The cavitand of formula II wherein R=$C_6H_{13}$ was synthesized with the following procedure

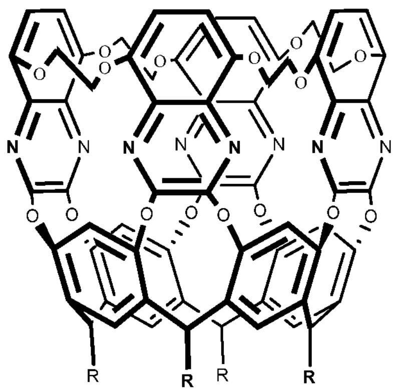

II

In a microwave test tube in an Argon atmosphere 190 mg (1,3.$10^{-4}$ mol; 1 eq.) of cavitand 4 are suspended in 5 ml of distilled DMF. The suspension is sonicated and 847 mg (2,6.$10^{-3}$ mol; 20 eq.) of anhydrous $Cs_2CO_3$ and 137

mg (0,76.$10^{-3}$ mol; 5,5 eq.) of ethylene glycol ditosylate are added. The reaction is conducted in a microwave reactor by irradiating the mixture at 100 W with magnetic agitation for 90 min, maintaining a temperature of 120°C. Once the reaction has taken place, the crude product is dried by means of a vacuum pump; the crude product is solubilized with 10 ml of dichloromethane and washed with $H_2O$ (3x10ml). The organic phase is recovered and dried. The product is purified using preparative flash column chromatography ($SiO_2$, eluent dichloromethane/Acetone:95/5). The product obtained is a yellow crystalline powder with a 70% yield.

**$^1$H-NMR** (300 MHz, $CDCl_3$): $\delta$ = 0.95 (t, 12H, *J* = 6.4 Hz, $\mathbf{CH_3}CH_2CH_2$), 1.20-1.62 (m, 32H, **$-CH_2-$**), 2.33 (bq, 8H $CH\mathbf{CH_2}CH_2$), 4.45-4.65 (m, 16H, $ArO\mathbf{CH_2CH_2}O$), 5.78 (t, 4H, *J* = 7.9 Hz, $\mathbf{CH}CH_2CH_2$), 6.70 (s, 8H, $Ar\mathbf{H_2}$), 7.4 (s, 4H, $Ar\mathbf{H}_{down}$), 8.30 (s, 4H, $Ar\mathbf{H}_{up}$)

**MALDI TOF-TOF:** calculated for $C_{92}H_{88}N_8O_{16}$ [M] m/z= 1560.6318, obtained m/z= 1560.6299 $[M]^+$

## Preparing the SPME fibres

The SPME fibres were made by immersing the silicon support of the fibres in an epoxy glue resistant to high temperatures and then in the finely pulverised solid cavitand.

Preparing the cartridges for P&T/dynamic headspace:

> The cartridges for dynamic headspace and *purge&trap* were made by filling normal glass cartridges with the appropriate amount of pulverised cavitand, previously sieved for having particle size in the range 60-80 mesh.

## SPME analysis

The SPME extraction was performed through the headspace technique by sampling the appropriate concentrations of analytes in gaseous phase at room temperature, for 15 min. The fibre was then desorbed in the gas chromatography injector at the temperature of 250°C for 2 minutes.

The GC-MS was conducted using a gas chromatograph HP 6890 Series Plus, Agilent Technologies (Milan, Italy), combined with a mass spectrometer MSD 5973 (Agilent Technologies) operating under the following conditions:

| Gas chromatograph HP 6890 Series Plus, Agilent Technologies (Milan, Italy) | |
|---|---|
| Column: | HP5-MS (l=30 m, i.d.=0.25 mm, d.f.=0.25 μm) |
| Vector gas: | Helium |
| Vector gas flow rate: | 1 ml/min |
| Injector temperature: | 250 °C |
| Temperature program: | 40°C for 8 minutes, gradient of 20°C/min up to 200°C |

| Mass spectrometer MSD 5973, Agilent Technologies | |
|---|---|
| Electron multiplier voltage: | 2,200 V |
| Ionization: | Electronic ionisation (70 eV) |
| Acquisition mode: | Time scheduled monitoring |
| Dwell time: | 30 ms |
| Monitored ions (Full Scan): | Range from 40 to 150 m/z |
| BTEX ions monitored (SIM) | |
| from 0,50 to 2,00 min | 78 m/z for benzene |
| from 2,00 to 4,00 min | 91 m/z for toluene |
| from 4,00 to 16,00 min | 91, 106 m/z for ethyl benzene and xylenes |
| Hydrocarbon ions monitored (SIM) | |
| from 0 to 2,00 min | 43, 57, 72 m/z for pentane |
| from 0 to 2,00 min | 43, 57, 72, 86 m/z for hexane |
| from 2,00 to 3,50 min | 43, 57, 71, 100 m/z for heptane |
| from 3,50 to 5,00 min | 43, 57, 85, 114 m/z for octane |
| from 5,00 to 16,00 min | 43, 57, 85, 128 m/z for nonane |

The acquisition and processing of the signals was performed using the software HP Chemstation (Agilent Technologies).

The method was validated by operating according to the Eurachem guidelines.

Limits of detection (LOD) and limits of quantitation (LOQ)

The limits were calculated through the calibration curves by means of the following expressions:

$$LOD = \frac{3\sigma_b}{b} \quad LOQ = \frac{10\sigma_b}{b}$$

where $\sigma_b$ is the standard deviation of the white calculated on five replicated measurements and b is the slope of the calibration curve.

Linearity

The calibration curves were created for each analyte in the ranges reported as follows:

| | |
|---|---|
| Benzene: | 3,48 - 348 ng/$m^3$ |
| Toluene: | 4,11 - 411 ng/m3 |
| Ethyl benzene: | 4,74 - 474 ng/m3 |
| m-xylene: | 4,73-473 ng/m3 |
| p-xylene: | 4,73-473 ng/m3 |
| o-xylene: | 4,73-473 ng/m3 |

The significance of the intercept (significance level = 5%) was established by performing the *t*-test. To check that the quadratic function was not significantly better than the linear one, the Mandel test was performed.

Precision

The precision was calculated in terms of repeatability and intermediate precision considering the relative standard deviation percentage (RSD%) on the two different concentration levels for each analyte in gaseous mixture, in the concentration range 37-400 ng/$m^3$.

The analysis of variance (ANOVA) was also applied to assess the absence of significant differences in the averages of the responses obtained on three different days.

Trueness

The trueness was assessed in terms of Recovery Rate (RR%) by adding the BTEX in the air to the concentrations reported below:

| | |
|---|---|
| Benzene: | 236 ng/$m^3$ |
| Toluene: | 278 ng/$m^3$ |
| Ethyl benzene: | 322 ng/$m^3$ |
| *m*-xylene: | 320 ng/$m^3$ |
| *p*-xylene: | 320 ng/$m^3$ |
| *o*-xylene: | 320 ng/$m^3$ |

Recovery

The recovery was then calculated according to the following expression:

$$RR\% = \frac{cobs}{cspike} \cdot 100$$

where $c_{obs}$ is the concentration determined in the samples analysed and $c_{spike}$ the concentration after addition.

**Selectivity studies**

In general, the cavitand QxBoxMe has higher complexation ability than QxBoxEt with respect to BTEX. The cavitand QxBoxEt reveals higher selectivity to xylenes, whereas the cavitand QxBoxMe shows excellent complexation ability to benzene. The different behaviour observed for the two receptors can be justified by the different size of the cavity. Analytes with a higher steric hindrance such as xylenes and ethyl benzene are withheld in the cavity of QxBoxEt more effectively than benzene and toluene: in fact, despite the ethylene spacer between the quinoxalines leading to a more rigid cavity, it is still too wide to allow ideal interaction with benzene. On the contrary, in the case of the QxBoxMe cavitand, the presence of a smaller spacer gives higher rigidity to the cavity promoting optimal interaction with benzene, the analyte to which, as shown in the histogram in Figure 4, the best selectivity is obtained.

The complexation abilities of the receptors were investigated further through consecutive desorptions of the materials. For all the cavitands, desorption of the analytes takes place at temperatures higher than 150°C, an indicator of the presence of strong interactions between receptor and analytes. For the cavitands QxBoxEt and QxBoxMe, complete release of BTEX takes place at the temperature of 250°C.

Subsequent selectivity tests regarded the sampling of aliphatic hydrocarbon mixtures, potential interferents in the environmental determination of BTEX. The tests conducted in the presence of such substances in concentrations 100 times higher than those of the analytes considered, highlighted how preliminary desorptions at low temperatures (50, 75°C) allow the complete release of such compounds, precluding all possible interference in the subsequent quantitative determination of BTEX (Fig. 5). Such behaviour can be explained through the absence of specific interactions ($\pi$-$\pi$ and CH-$\pi$) with the cavity of the receptors, therefore desorptions at low temperatures are sufficient to allow the complete release of such substances, only withheld by means of nonspecific interactions.

The performance levels of the cavitands QxBoxEt and QxBoxMe were finally compared with those of commercially available materials such as the SPME fibres 75$\mu$m Carboxen-PDMS and 2cm-50/30$\mu$m PDMS-Carboxen-DVB. The use of these fibres was dictated by the presence of some analogies with the QxBox fibres. They all operate through the adsorption mechanism and are suitable for the extraction of BTEX both by size and due to the establishment of $\pi$-$\pi$ type interactions between analytes and the stationary phase. CAR-PDMS is one of the most commonly used fibres for the determination of BTEX since it is suitable for extracting medium-low polarity and small compounds, whereas DVB-CAR-PDMS can be effectively used to extract BTEX for establishing $\pi$-$\pi$ type interactions between the aromatic rings of the DVB phase and the aromatic rings of the analytes. As it is clear from the results obtained (see Table 1), the QxBox fibres show substantially higher extraction abilities than those obtained with commercial fibres.

**Table 1.** Detection limits (ng/$m^3$) of cavitands and of other techniques reported in literature.

| The extraction time is indicated in brackets. | | | | | | |
|---|---|---|---|---|---|---|
| | **EtGxBox** (15 min) | **MeQxBox** (15 min) | **DVB-CAR-PDMS** (15 min) | **CAR-PDMS** (15 min) | **RADIELLO** (24 h) | **OPTIC FIBRE*** (25 min) |
| benzene | 2.8 | 0,40 | 17,1 | 16,4 | 290 | 1,6 |
| toluene | 0.08 | 0,34 | 2,1 | 4,4 | 90 | 1,5 |
| ethyl benzene | 0.01 | 0,51 | 4,8 | 14,2 | 40 | 1,2 |
| *m*-xylene | 0.002 | 1,17 | 6,1 | 18,4 | 70 | 1,3 |
| *p*-xylene: | 0.001 | 0,56 | 6,1 | 18,4 | 80 | 1,7 |
| *o*-xylene: | 0.1 | 1,02 | 14,3 | 24,6 | 10 | 2,0 |
| * L.I.B. Silva, T.A.P. Santos, A.C. Duarte, Talanta, 78 (2009) 548 | | | | | | |

The extraction ability of the QxBoxMe fibre was finally verified by performing the sampling in real environmental conditions, hence analysing a gaseous mixture in which benzene is present in a lower concentration than other aromatic hydrocarbons.

As can be observed from the histogram reported in Figure 6, the QxBoxMe cavitand shows higher selectivity to benzene despite the greater presence in the air of the other BTEX.

Finally, the SPME-GC-MS method was validated for the determination in the air of BTEX, showing excellent performance levels both in terms of limits of detection (LOD) and quantitation (LOQ), linearity, precision and trueness.

In particular, as reported in Table 1, the cavitands allow detection limits of a few ng/$m^3$ to be reached, hence enabling the presence of trace levels of analytes to be detected.

As highlighted by the results of Table 1, the enrichment abilities of the cavitands QxBoxEt and QxBoxMe are

substantially higher both with respect to other techniques currently used for the quantitation of BTEX (Radiello), and to data from literature. In fact, the cavitands allow low concentration values to be detected more quickly, thanks to specific host-guest interactions hence promoting the selective sampling of such compounds. The performance levels reached therefore allow the possible use of the cavitands as sensitive materials for the assessment of short term exposure limits during work (TLV-STEL), which cannot be measured through techniques based on the use of passive samplers, such as Radiello, which requires long exposure times. Finally, it can be observed that the cavitand QxBoxMe has a benzene detection limit of one seventh of that obtained with the cavitand QxBoxEt, which however has better limits for xylene and toluene. The data is in line with the results deriving from selectivity studies confirming the hypothesis that the presence of the methyl bridge between the quinoxaline units promotes better closure of the cavity than the presence of an ethyl bridge, and therefore reduced cavitand-analyte interaction for larger molecules than benzene.

Excellent results were also obtained in terms of response linearity (verified for all analytes in two orders of magnitude), precision (with CV < 10% even on different days), trueness (with recovery rate between 99 $\pm$ 1 and 103 $\pm$ 1%, n=3).

The affinity of QxBoxMe and QxBoxEt to aromatic hydrocarbons was also studied through single crystal x-ray diffraction. The geometric descriptions of the two cavitands and the structures of their coordination complexes with benzene and toluene are reported below.

## Solid state complexation studies

The two receptors were studied in the solid state through single crystal x-ray diffraction. This enabled the dimensions of the cavity in the two receptors to be measured and the number, type and geometry of the interactions with the aromatic analytes to be established.

### QxBoxEt

The depth of the cavity is about 8.1 Å, calculated considering the distance between the through plane for the group of atoms C7 (A, B, C and D) and C19-C20 (A, B, C and D). The cavity has a rectangular opening of about 10x7 Å. The four quinoxaline walls are inclined by 87.09(3), 80.17(3), 86.37(3) and 81.16(3) degrees for groups A, B, C and D with respect to the through plane for the four groups of atoms O1 and O2 (Figure 7).

In the coordination complexes QxBoxEt@benzene and QxBoxEt@toluene the position of the analyte within the cavitand is due to the presence of $\pi$-$\pi$ and CH-$\pi$ interactions with the aromatic cavity. In particular, in the case of toluene, the orientation is different due to the presence of methyl which is disordered in two equivalent geometric positions (see Figure 8). This disorder is possible thanks to the dimensions and geometry of the cavity.

### QxBoxMe

The depth of the cavity (8.06 Å) was calculated in the same way as for QxBoxEt; the main difference between the two receptors lies in the shape of the cavity which in this case is a rhomboid rather than a rectangle (dimensions: 8x12 Å approx.). The quinoxaline walls are inclined by 77.21(3), 81.94(4), 81.27(3) and 84.30(4) degrees for groups A, B, C and D, with respect to the plane that passes through the atoms O1 and O2 (Figure 9).

Also in this case, in the coordination complexes QxBoxMe@benzene and QxBoxMe@toluene, the position of the analyte within the cavitand is due to the presence of $\pi$-$\pi$ and CH-$\pi$ interactions with the aromatic cavity (Figure 10).

The dimensions of the QxBoxMe cavity are more complementary to benzene with respect to those of QxBoxEt. The new geometry and smaller space available also affect the position of the toluene, which has a single orientation without disorder phenomena.

## Claims

1. A compound according to the following general formula:

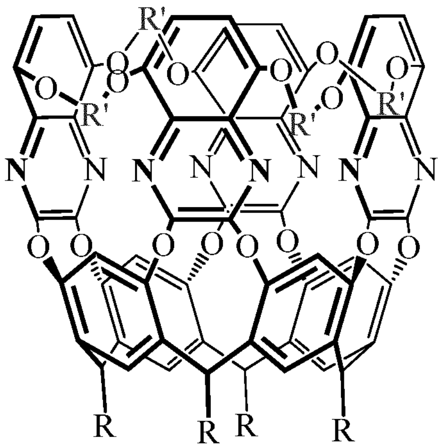

wherein R' is chosen from $-CH_2-$ and $-CH_2-CH_2-$;
R is a $C_1$-$C_{20}$ linear or branched alkyl chain, not substituted or substituted with a group selected from: an olefin, acetylenic, alcoholic, azidic, phosphonic or acid group.

**2.** The compound according to claim 1, wherein R is a linear or branched alkyl chain with 6 carbon atoms.

**3.** The compound according to claim 1 or 2, wherein the group substituting the alkyl chain R is at an end position of the chain.

**4.** The compound according to any one of claims from 1 to 3, wherein R' is $-CH_2-$ and R is a linear alkyl chain with 6 carbon atoms.

**5.** The compound according to any one of claims from 1 to 3, wherein R' is $-CH_2-CH_2-$ and R is a linear alkyl chain with 6 carbon atoms.

**6.** The use of the compound according to any one of claims from 1 to 5, for the preparation of fibres, SPME, to be used for the selective microextraction of pollutants, preferably benzene, toluene, ethyl benzene, *o-, m-*, *p*-xylene, from the air.

**7.** The use of the compound according to any one of claims from 1 to 5, for the preparation of cartridges for dynamic headspace or *purge&trap* to be used for recognising and adsorbing pollutants, preferably benzene, toluene, ethyl benzene, *o-, m-*, *p*-xylene, from the air.

**8.** The use of the compound according to any one of claims from 1 to 5 for monitoring atmospheric pollution, preferably caused by benzene, toluene, ethyl benzene, *o*-, *m*-, *p*-xylene.

**9.** An SPME fibre prepared and/or coated with the compound of formula (I) according to any one of claims from 1 to 5.

**10.** The cartridge for dynamic headspace or *purge&trap* manufactured with the compound according to any one of claims from 1 to 5.

**11.** The use of the fibre according to claim 9 or the cartridge according to claim 10 for the selective determination, through adsorption or pre-concentration, of pollutants preferably chosen from benzene, toluene, ethyl benzene, o-, m-, p-xylene in the air.

**12.** A method for determining and monitoring the concentration of atmospheric pollutants, preferably chosen from benzene, toluene, ethyl benzene, o-, m-, p-xylene, in the air, comprising the selective adsorption of said pollutants on a fibre or cartridge prepared/coated with a compound according to any one of claims from 1 to 5 and subsequent desorption and analysis of the pollutants through chromatographic techniques, preferably GC-MS.

## Patentansprüche

1. Eine Verbindung der folgenden allgemeinen Formel:

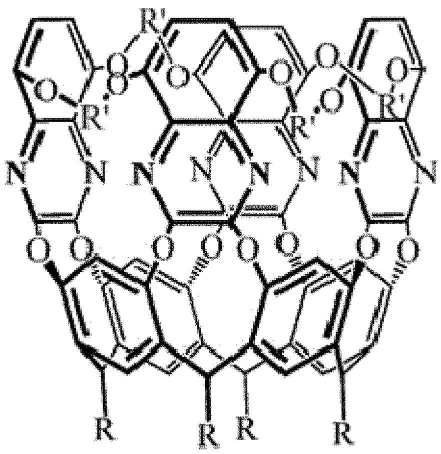

Wobei R' für eine -CH2- oder eine -CH2-CH2- Brücke steht; R steht für eine lineare oder verzweigte C1-C20 Alkylkette, unsubstituiert oder substituiert mit einer der folgenden Funktionen: Doppel- oder Dreifachbindung, Alkohol, Azid, Phosphon- oder Carbonsäure.

2. Eine Verbindung nach Anspruch 1, wobei es sich bei R um eine lineare oder verzweigte Alkylkette mit 6 Kohlenstoffatomen handelt.

3. Eine Verbindung nach Anspruch 1 oder 2, bei welcher das Ende der Alkylkette R funktionalisiert ist.

4. Eine Verbindung nach einem der Ansprüche 1 bis 3, bei welcher R für -CH2- und R' für eine lineare Alkylkette mit 6 Kohlenstoffatomen steht.

5. Eine Verbindung nach einem der Ansprüche 1 bis 3, bei welcher R für -CH2-CH2- und R' für eine lineare Alkylkette mit 6 Kohlenstoffatomen steht.

6. Die Verwendung eine Verbindung, beschrieben in einem den Ansprüchen 1 bis 5, für die Herstellung von Fasern zur Festphasenmikroextraktion von Schadstoffen, insbesondere von Benzol, Toluol, Ethylbenzol und o-, m- und p-Xylol, aus der Luft.

7. Die Verwendung eine Verbindung, beschrieben in einem den Ansprüchen 1 bis 5, für die Herstellung von Kartuschen für die dynamische Headspace- oder Purge&Trap-Technik, Zwecks der Erkennung und Adsorption von Schadstoffen, insbesondere von Benzol, Toluol, Ethylbenzol und o-, m- und p-Xylol, aus der Luft.

8. Die Verwendung eine Verbindung, beschrieben in einem den Ansprüchen 1 bis 5, für die Luftschadstoffüberwachung, insbesondere verursacht durch Benzol, Toluol, Ethylbenzol oder o-, m-oder p-Xylol.

9. Eine Festphasenmikroextraktionsfaser, hergestellt oder beschichtet mit einer Verbindung mit der allgemeinen Formel (I), nach einem der Ansprüche 1 bis 5.

10. Eine Kartusche für die dynamische Headspace- oder Purge&Trap-Technik, hergestellt mit einer Verbindung erwähnt in einem der Ansprüche 1 bis 5.

11. Die Verwendung eine Faser, wie erwähnt in Anspruch 9, oder einer Kartusche, wie erwähnt in Anspruch 10, für die selektive Bestimmung von Schadstoffen, insbesondere von Benzol, Toluol, Ethylbenzol oder o-, m- oder p-Xylol, durch Adsorption oder Vorkonzentration aus Luft.

12. Eine Methode zur Bestimmung und Quantifizierung von Luftschadstoffen, insbesondere von Benzol, Toluol, Ethylbenzol und o-, m- und p-Xylol, bestehend aus der Adsorption der genannten Schadstoffe durch eine Faser oder

eine Kartusche, hergestellt und/oder beschichtet mit einer Verbindung erwähnt in einem der Ansprüche 1 bis 5, gefolgt von der Austreibung und chromatographischen Analyse, insbesondere mittels GC-MS.

## Revendications

**1.** Un composé ayant la formule générale ci-dessous :

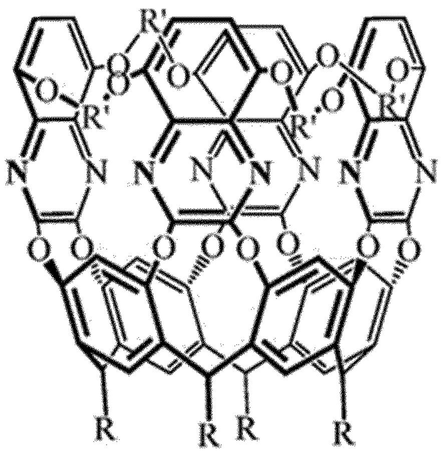

où R' est choisi parmi $-CH_2-$et $-CH_2-CH_2-$;
R est une chaine aliphatique $C_1-C_{20}$ linéaire ou ramifiée, non substituée ou substituée avec l'un des groupes choisis parmi : une oléfine, un groupe acétylénique, alcoolique, azidique, phosphonique ou acide.

**2.** Le composé selon la revendication 1, où R est une chaine alkyle linéaire ou ramifiée, à 6 atomes de carbones.

**3.** Le composé selon les revendications 1 ou 2, où le groupe substituant la chaine alkyle R est à la fin de la chaine.

**4.** Le composé selon l'une des revendications 1 à 3, où R' est $-CH_2-$et R est une chaine alkyle linéaire à 6 atomes de carbone.

**5.** Le composé selon l'une des revendications 1 à 3, où R' est $-CH_2-CH_2-$ et R est une chaine alkyle linéaire à 6 atomes de carbone.

**6.** L'utilisation du composé selon l'une des revendications 1 à 5, pour la préparation de fibres SPME, afin d'être utilisée pour la micro extraction de polluants, préférentiellement benzène, toluène, ethylbenzène, *o-, m-, p*-xylène, dans l'air.

**7.** L'utilisation du composé selon l'une des revendications 1 à 5, pour la préparation de cartouche pour espace de tête dynamique ou *purge & trap,* utilisés pour la reconnaissance et l'adsorption de polluants, préférentiellement benzène, toluène, ethylbenzène, *o-, m-, p*-xylène.

**8.** L'utilisation du composé selon l'une des revendications 1 à 5, pour le suivi de la pollution atmosphérique, préférentiellement causée par benzène, toluène, ethylbenzène, *o-, m-, p*-xylène.

**9.** Une fibre SPME confectionnée et/ou recouverte avec le composé de formule (I) selon l'une des revendications 1 à 5.

**10.** La cartouche pour espace de tête dynamique ou *purge & trap,* confectionnée avec le composé selon les revendications 1 à 5.

**11.** L'utilisation de la fibre selon la revendication 9 ou de la cartouche selon la revendication 10, pour la reconnaissance sélective, par adsorption ou préconcentration, de polluants préférentiellement choisis parmi benzène, toluène, ethylbenzène, o-, m-, p-xylène, dans l'air.

**12.** Une méthode pour déterminer et contrôler la concentration de polluants dans l'air atmosphérique, préférentiellement

choisis parmi benzène, toluène, ethylbenzène, o-, *m-, p*-xylène, comprenant l'adsorption sélective desdits polluants sur une fibre ou une cartouche préparée/recouverte avec un composé selon l'une des revendications 1 à 5, et successivement désorption et analyse des polluants par méthode chromatographique, préférentiellement GC-MS.

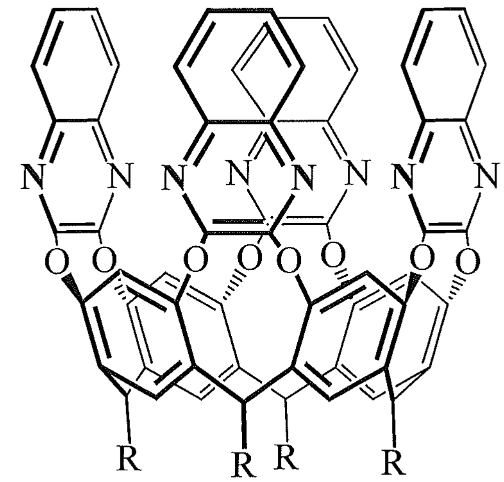

Fig. 1

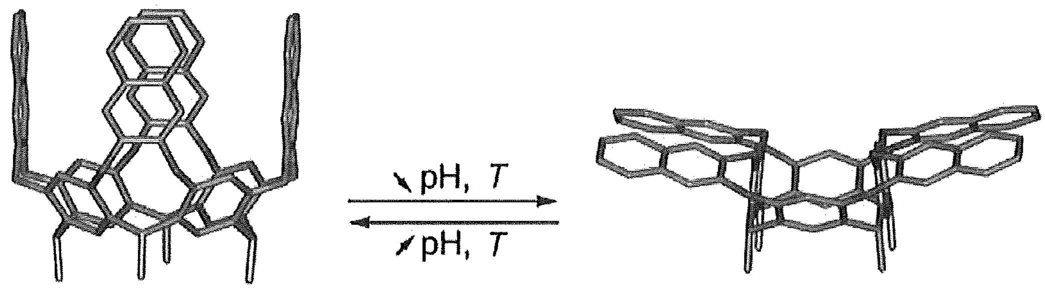


Vase ($C_{4v}$)    Kite ($C_{2v}$)

**Fig. 2**

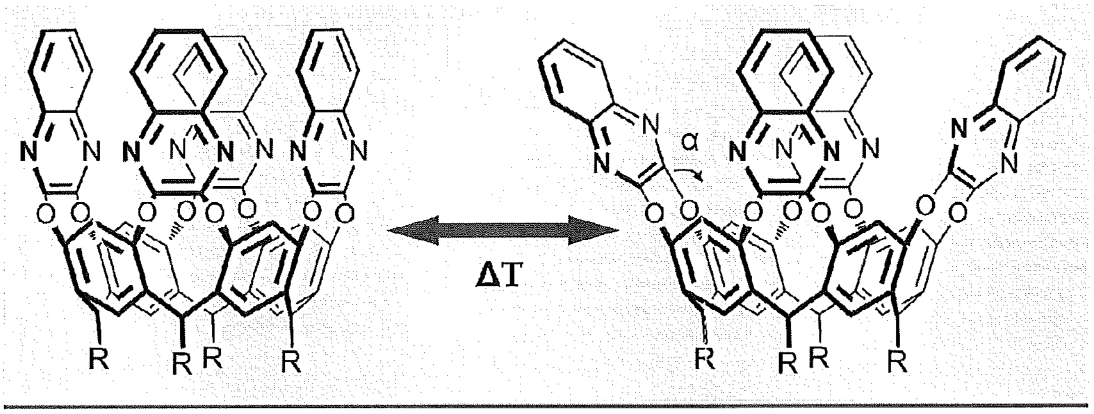


α = The medium opening angle 16°

Fig. 3

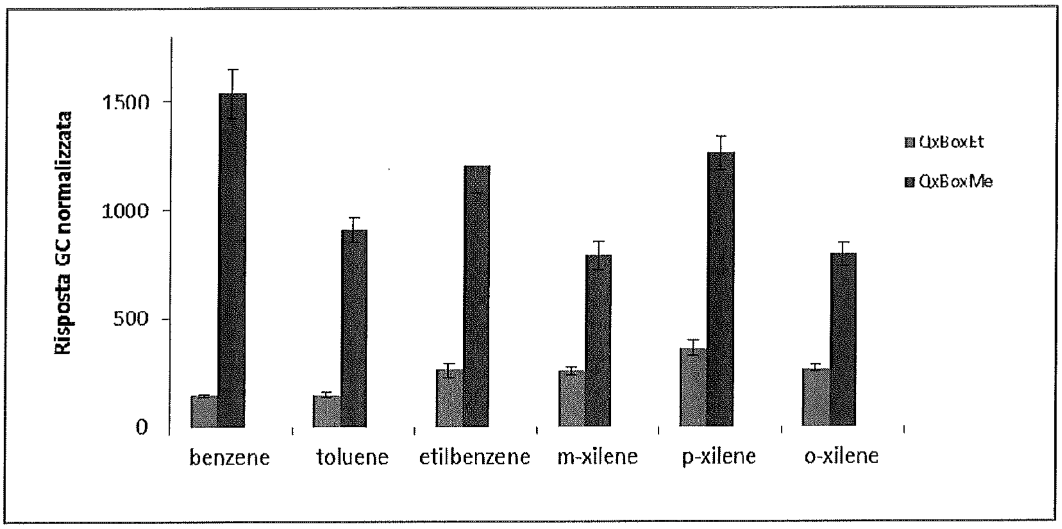
Risposta GC normalizzata
1500
1000
500
0
benzene
toluene
etilbenzene
m-xilene
p-xilene
o-xilene
QxBoxEt
QxBoxMe

**Fig. 4**

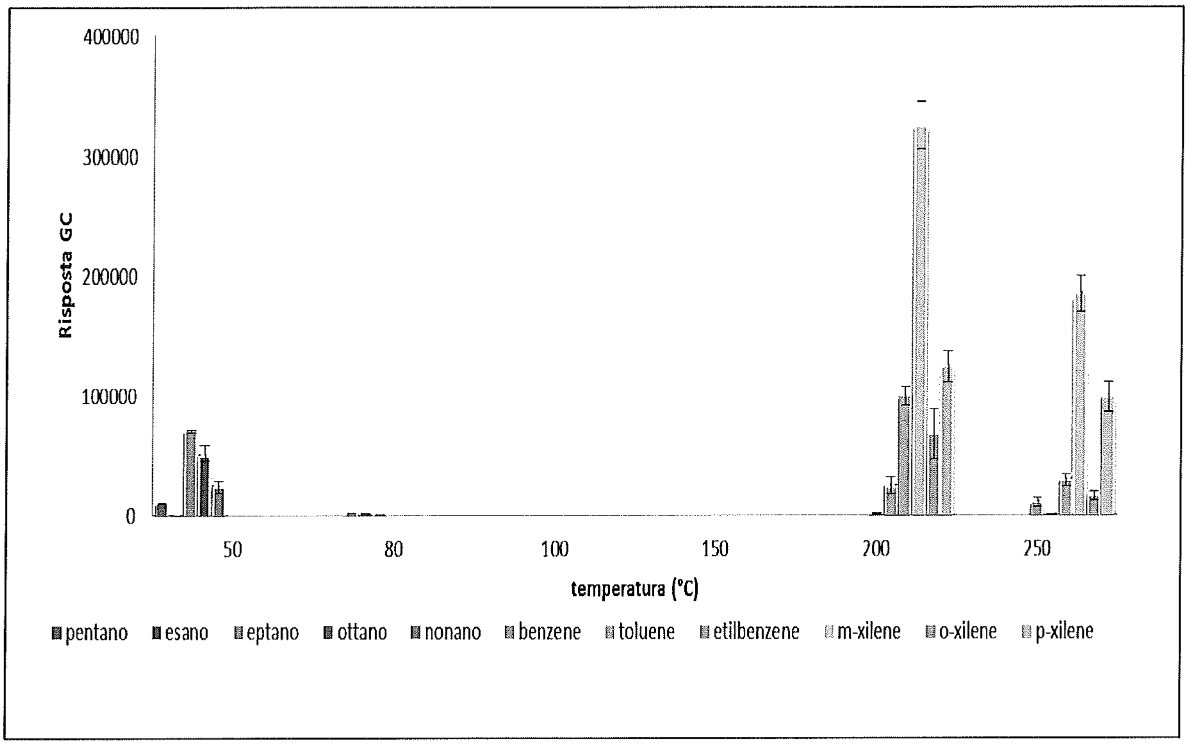
400000
300000
200000
100000
0
Risposta GC
50
80
100
150
200
250
temperatura (°C)
pentano
esano
eptano
ottano
nonano
benzene
toluene
etilbenzene
m-xilene
o-xilene
p-xilene

Fig. 5

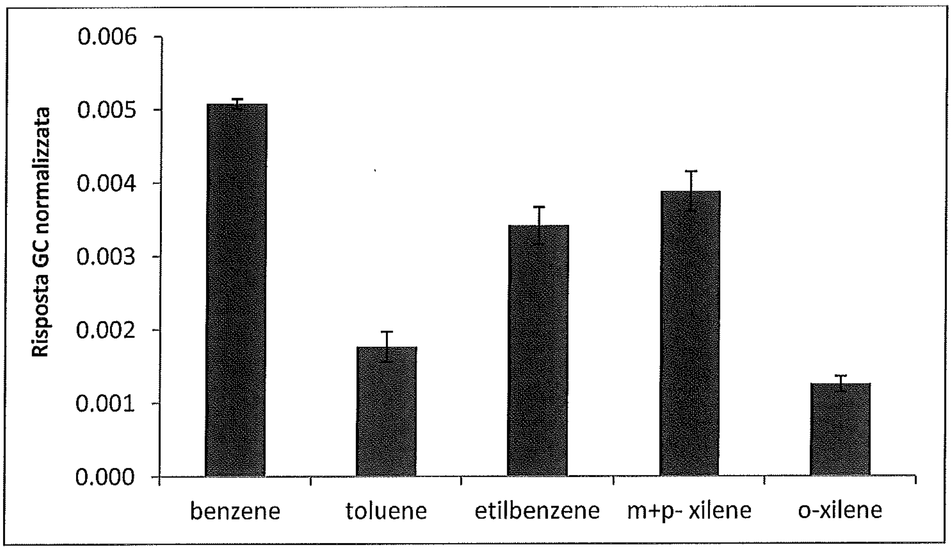
0.006
0.005
0.004
0.003
0.002
0.001
0.000
Risposta GC normalizzata
benzene
toluene
etilbenzene
m+p- xilene
o-xilene

Fig. 6

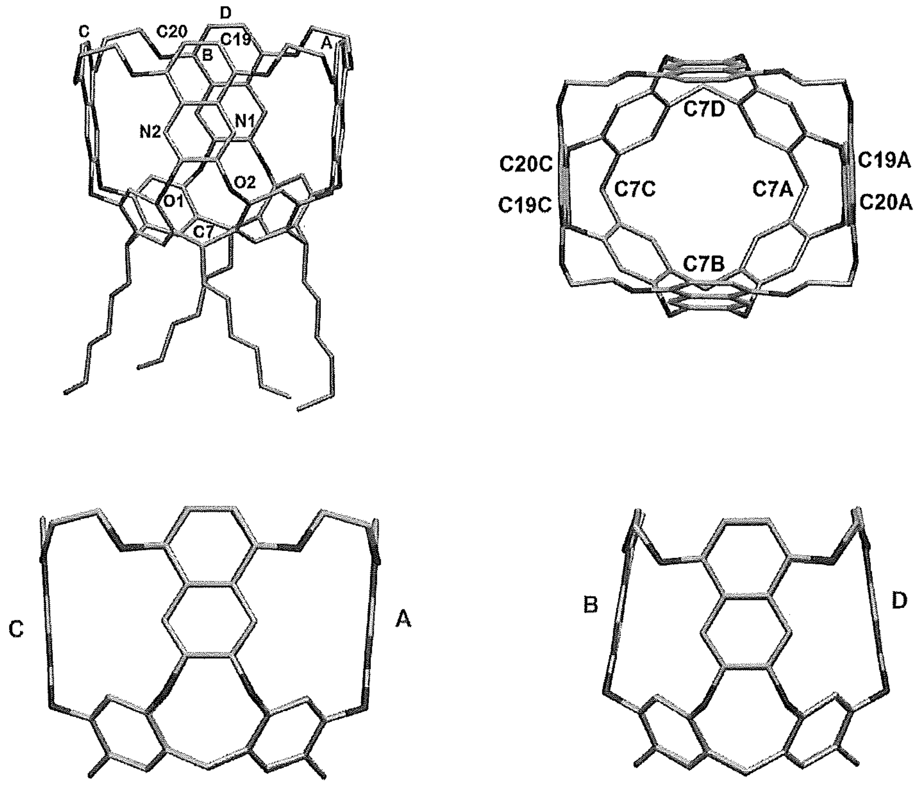

Fig. 7

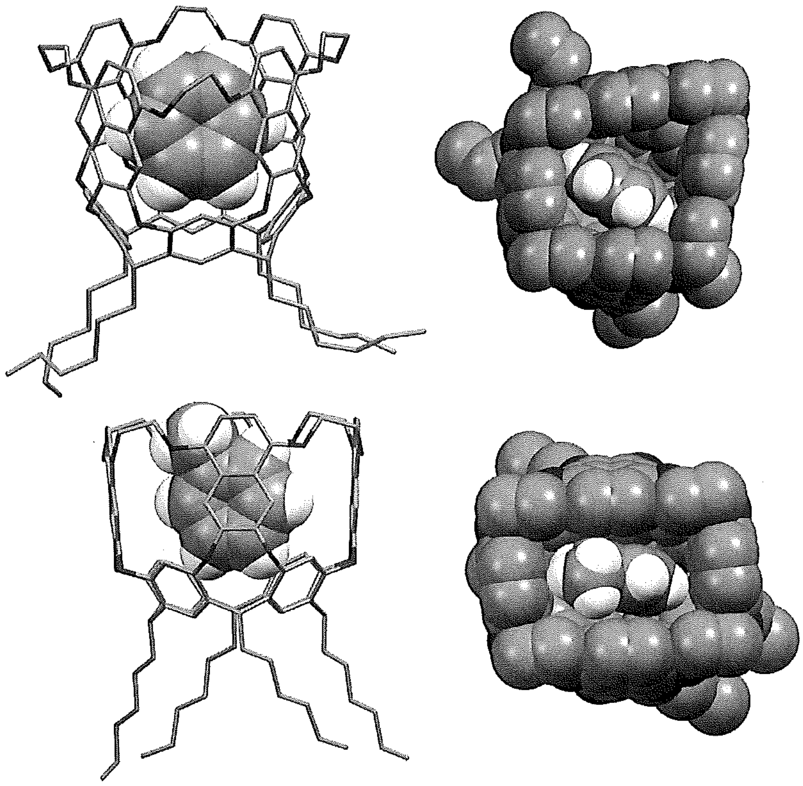

Fig. 8

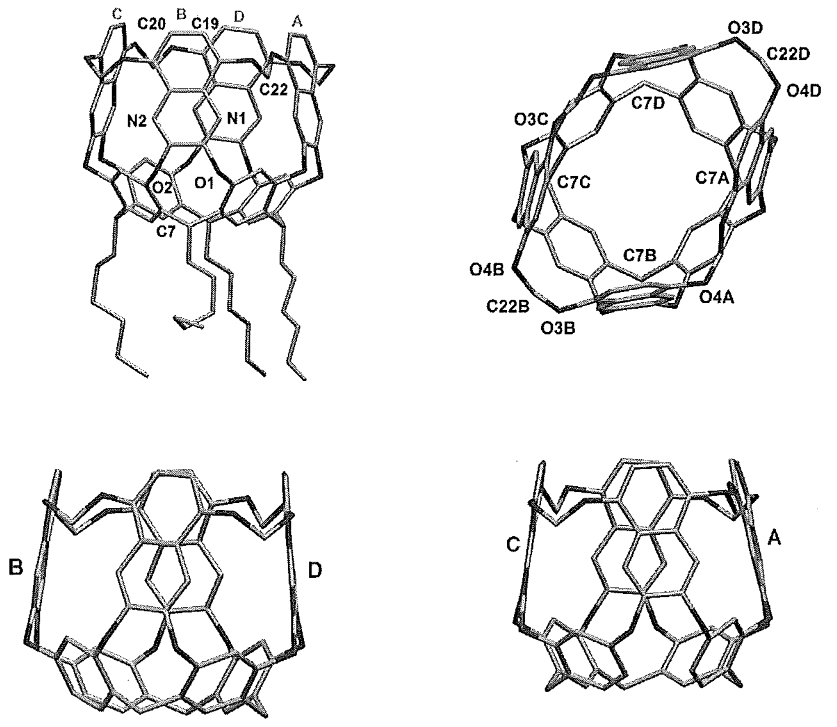

Fig. 9

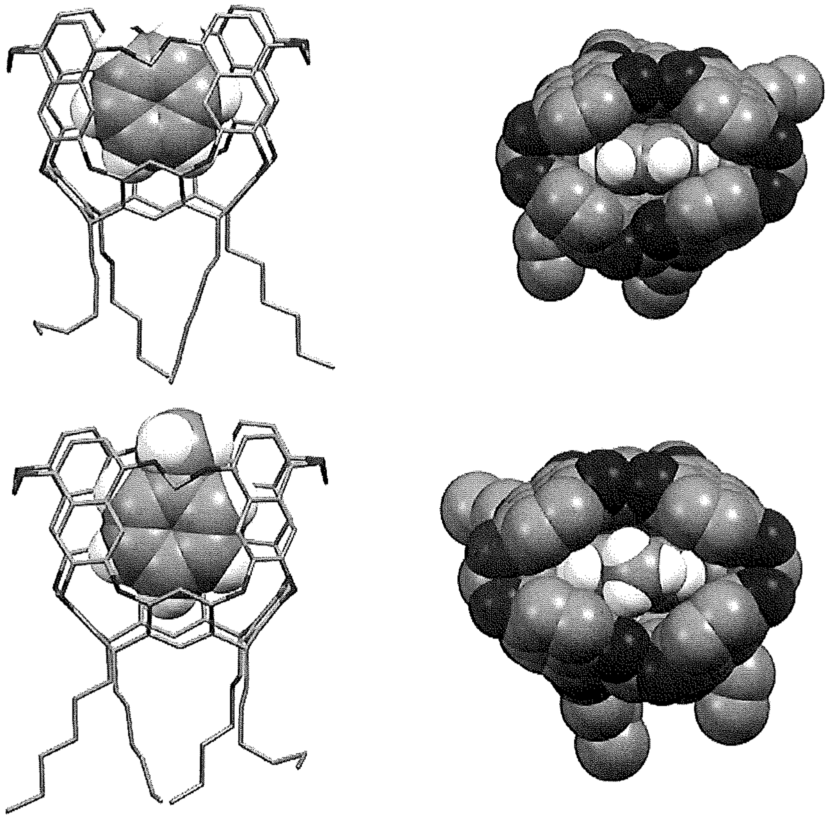

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Volatile Organic Compounds in Indoor Air: A Review of Concentrations Measured in North America Since 1990. **A.T. HODGSON ; H. LEVIN.** Off. J. Eur. Communities, L 313/12. Lawrence Berkeley National Laboratory, 2003, 31
- **S. ZAMPOLLI ; I. ELMI ; F. MANCARELLA ; P. BETTI ; E. DALCANALE ; G.C. CARDINALI ; M. SEVERI.** *Sensors and Actuators B,* 2009, vol. 141, 322-328
- **S. ZAMPOLLI ; P. BETTI ; I. ELMI ; E. DALCANALE.** *Chem. Commun.,* 2007
- **P. SONCINI ; S. BONSIGNORE ; E. DALCANALE ; F. UGOZZOLI.** *J. Org. Chem.,* 1992, vol. 57, 4608
- **M. VINCENTI ; E. DALCANALE ; P. SONCINI ; G. GUGLIELMETTI.** *J. Am. Chem. Soc.,* 1990, vol. 112, 445
- **M. VINCENTI ; E. PELIZZETTI ; E. DALCANALE ; P. SONCINI.** *Pure Appl. Chem.,* 1993, vol. 65, 1507
- **M. VINCENTI ; E. DALCANALE.** J. Chem. Soc. Perkin Trans, 1995, vol. 2, 1069
- **E. DALCANALE ; G. COSTANTINI ; P. SONCINI.** *J. Incl. Phenom. Mol Recognit. Chem.,* 1992, vol. 13, 87
- **E. DALCANALE ; J. HARTMANN.** *Sens. Actuators B,* 1995, vol. 24, 39
- **F. BIANCHI ; R. PINALLI ; F. UGOZZOLI ; S. SPERA ; M. CARERI ; E. DALCANALE.** *New J. Chem.,* 2003, vol. 27, 502
- **E.B. FERESENBET ; E. DALCANALE ; C. DULCEY ; D.K. SHENOY.** *Sens. Actuators B,* 2004, vol. 97, 211
- **S. ZAMPOLLI ; P. BETTI ; I. ELMI ; E. DALCANALE.** *Chem. Commun.,* 2007, 2790-2792
- **F. BIANCHI ; M. MATTAROZZI ; P. BETTI ; F. BISCEGLIE ; M. CARERI ; A. MANGIA ; L. SIDISKY ; S. ONGARATO ; E. DALCANALE.** *Anal. Chem.,* 2008, vol. 80, 6423
- **L. SHIRTCLIFF ; H. XU ; F. DIEDERICH.** *Eur. J. Org. Chem.,* 2010, 846
- **L. M. TUNSTAD ; J. A. TUCKER ; E. DALCANALE ; J. WEISER ; J. A. BRYANT ; J. C. SHERMAN ; R. C. HEGELSON ; C. B. KNOBLER ; D.J. CRAM.** *J. Org. Chem.,* 1989, vol. 54, 1305-1312
- **S. OGUCHI.** *Bull. Chem. Soc. Jp.,* 1968, vol. 41, 980-987
- **L.I.B. SILVA ; T.A.P. SANTOS ; A.C. DUARTE.** *Talanta,* 2009, vol. 78, 548